# EUROPEAN PATENT APPLICATION

(11) **EP 4 681 651 A1**
(43) Date of publication of application: **21.01.2026**
(21) Application number: 24189388.2
(22) Date of filing: 18.07.2024
(51) Int. Cl.: A61B 6/50, A61B 6/00, A61B 8/08, A61B 8/12, G06T 7/30

(54) **RENAL DENERVATION TREATMENT GUIDANCE USING HEMODYNAMIC CO-REGISTRATION AND ASSOCIATED SYSTEMS, DEVICES, METHODS**

(71) Applicant: Koninklijke Philips N.V., 5656 AG Eindhoven (NL)
(72) Inventor: CEZO, James David, Eindhoven (NL); CHEN, Sara Rose, Eindhoven (NL)
(74) Representative: Philips Intellectual Property & Standards

(57) **Abstract**

An apparatus includes a processor in communication with an intravascular catheter or guidewire. The processor controls the catheter or guidewire to obtain intravascular sensor measurements within a renal artery of a patient, and calculates hemodynamic values based on the intravascular sensor measurements. The hemodynamic values are associated with a suitability of the patient for a renal denervation treatment or an expected responsiveness of the patient to the treatment. The processor also performs co-registration between the hemodynamic values and a first X-ray image of the renal artery obtained with a contrast fluid inside the renal artery, and outputs, to a display, a screen display configured to guide a user regarding the renal denervation treatment. The screen display includes the first X-ray image of the renal artery and a representation of at least one hemodynamic value positioned proximate to at least one corresponding location along the length of the renal artery.

## Description

### FIELD

The subject matter described herein relates to systems, devices, and methods for co-registration of renal angiography with intravascular hemodynamic metrics during a renal denervation (RDN) procedure. This RDN hemodynamics co-registration system has particular but not exclusive utility for improving speed and outcomes for renal denervation procedures.

### BACKGROUND

Renal denervation (RDN) is a potentially promising procedure for the treatment of hypertension that cannot adequately be treated with anti-hypertensive medications. Studies demonstrate effectiveness of the treatment with a response rate of about 2/3 of patients gaining a decrease of systolic blood pressure of 5 mm Hg or more. However, studies have also shown varying levels of efficacy, with some portion of the population (currently estimated at approximately 30%) showing no response or negative response to the procedure. This demographic of non-responders may present as such for reasons that may stem from a combination of physiological factors and/or inadequately administered therapy.

The information included in this Background section of the specification, including any references cited herein and any description or discussion thereof, is included for technical reference purposes only and is not to be regarded as subject matter by which the scope of the disclosure is to be bound.

### SUMMARY

Disclosed is an RDN hemodynamics co-registration system which enables a clinician to predict which treatment locations are likely to show favorable response to RDN treatment, and/or to determine the success or completion of an RDN treatment in progress. The present disclosure involves projecting co-registered hemodynamic data onto fluoroscopic images of the renal artery during an RDN procedure. Currently, renal denervation (RDN) procedures are performed intravascularly under fluoroscopic guidance, with little information available about the correct locations to perform the RDN procedure or the tissues adjacent to the artery. Invasive intravascular measurements, such as invasive pulse wave velocity (iPWV), can be used to evaluate the chances of success with the RDN procedure, and the variation of these parameters with location along the renal artery may impact the efficacy of RDN procedures and can thus help guide the user where they should or should not use the RDN treatment device(s). The RDN hemodynamics co-registration system disclosed herein has particular, but not exclusive, utility for determining the suitability of locations for, the expected success of, and/or the completion status of, renal denervation procedures.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the RDN hemodynamics co-registration system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
**Figure 1A** is a schematic, diagrammatic representation, in block diagram form, of an example intravascular sensing system, according to aspects of the present disclosure.
**Figure 1B** is a schematic, diagrammatic representation, in block diagram form, of an example intravascular sensing system, according to aspects of the present disclosure.
**Figure 2** is a schematic diagram of a processor circuit, according to aspects of the present disclosure.
**Figure 3** is a schematic, diagrammatic representation, of an intravascular sensing guidewire being pulled back through the left renal artery of the left kidney, according to aspects of the present disclosure.
**Figure 4** is a schematic, diagrammatic representation of an X-ray image, with contrast, of the renal arterial tree, according to aspects of the present disclosure.
**Figure 5** is a schematic, diagrammatic representation, in block diagram form, of an example hemodynamic parameter measurement process, according to aspects of the present disclosure.
**Figure 6A** is a schematic, diagrammatic representation of the co-registration of hemodynamic parameter measurements with the angiographic roadmap image of the renal arterial tree, according to aspects of the present disclosure.
**Figure 6B** is a schematic, diagrammatic representation of the co-registration of raw sensor measurements with the angiographic roadmap image of the renal arterial tree, according to aspects of the present disclosure.
**Figure 7** is a co-registration screen display of an example RDN hemodynamics co-registration system, according to aspects of the present disclosure.
**Figure 8** is a co-registration screen display of an example RDN hemodynamics co-registration system, according to aspects of the present disclosure.
**Figure 9** is a co-registration screen display of an example RDN hemodynamics co-registration system, according to aspects of the present disclosure.
**Figure 10** is a co-registration screen display of an example RDN hemodynamics co-registration system, according to aspects of the present disclosure.
**Figure 11** is a graph showing a relationship between RDN outcomes and the measured value of iPWV, according to aspects of the present disclosure.
**Figure 12** is a schematic, diagrammatic representation, in flow diagram form, of an example RDN hemodynamics co-registration method, according to aspects of the present disclosure.
**Figure 13** is a schematic, diagrammatic representation, in flow diagram form, of an example RDN hemodynamics co-registration method, according to aspects of the present disclosure.
**Figure 14** is a schematic, diagrammatic representation, in block diagram form, of an example RDN hemodynamics co-registration process, according to aspects of the present disclosure.
**Figure 15** is a schematic, diagrammatic representation, in block diagram form, of an example RDN hemodynamics co-registration process, according to aspects of the present disclosure.
**Figure 16** is a schematic, diagrammatic representation of the renal vasculature of a patient, with a renal denervation treatment device in the left renal artery of the left kidney, according to aspects of the present disclosure.

### DETAILED DESCRIPTION

In accordance with at least one aspect of the present disclosure, an RDN hemodynamics co-registration system is provided which enables a clinician to predict which treatment locations are likely to show favorable response to RDN treatment, and/or to determine the success or completion of an RDN treatment in progress.

Renal denervation (RDN) is an emerging treatment for high blood pressure that cannot adequately be treated with anti-hypertensive medications. Studies demonstrate effectiveness of the treatment with a response rate of about 2/3 of patients gaining a decrease of systolic blood pressure of 5 mm Hg or more. Hemodynamic parameters are under investigation to determine whether they can help predict patients more likely to respond to renal denervation. These can include renal wave speed and renal resistance. A combination of factors may improve predictive power. Non-invasive measures such as baseline blood pressure are not very conclusive. However, one study reports invasive pulse wave velocity (iPWV) is an independent predictor for blood pressure response after RDN, using a cutoff value of 14.4 m/s. Patients with iPWV below this cutoff had a stronger response. Hemodynamic parameters such as iPWV may not be directly measurable, but are rather calculated from directly measurable variables such as pressure and flow velocity. Other hemodynamic parameters that may be calculated and co-registered include, but are not limited to, heart rate, volumetric flow rate, blood flow velocity, vascular resistance or impedance, pulsatility, arterial stiffness, renal resistance, and changes in arterial diameter.

Currently, before and during the RDN procedure, clinicians do not have visibility to hemodynamic data that might impact the patient's response to RDN. The RDN hemodynamics co-registration system of the present disclosure brings relevant hemodynamic information to the angiographic display, for operators to easily review when embarking on a renal denervation procedure.

Before the RDN treatment is applied, the clinician can use a doppler wire or pressure-flow combo wire to assess flow velocity in the renal artery. This flow velocity, renal arterial resistance, renal arterial resistance reserve, a multi-factor index, and/or changes in these measures before and after treatment can be displayed on a screen. Alternatively, changes in these measures before and after a change in physiological state could be displayed on the screen.

This can be done similarly to the way instantaneous wave free ratio (iFR) is displayed in systems assessing the coronary arteries. The display for prediction of RDN responders may for example place the location of the index is directly on the location of the angiogram where the index was measured. The index itself, iPWV, renal resistance, or other, can be provided on screen with guidance as to interpretation of the value, either association with positive treatment response, minimal treatment response, or no treatment response. A graphic may also demonstrate variability of the measure across the length of the artery. The output could also be overlayed onto existing correlation graphics from publications as background for the prediction.

In another aspect, the hemodynamics are assessed before and after a physiological state change using pharmaceuticals, stimulation of the renal nerve bundle, mechanical stimulation of the renal artery, or sympathetic nervous system stimulation at another anatomic location (carotid bodies for example). In this aspect, the hemodynamic parameter can be assessed at a baseline condition and compared to the same hemodynamic parameter after the physiological state change. This comparison may include a ratio between the baseline and physiological state change, a subtraction or addition of the two measurements, or multiplication of the measurements with other physiological parameters of the patient such as arterial bloop pressure, mean BP, systolic BP, or diastolic BP, or multiplication of the measurements with other patient data such as BMI, age, weight, or a combination of any of these comparisons. This comparison between hemodynamic measures can then be displayed adjacent to the angiography of the renal arterial tree.

The present disclosure brings intravascular sensing data, and hemodynamic parameters calculated from it, onto the fluoroscopic or angiographic view that the physician is using to guide an RDN procedure. The location of the renal nerve in relation to the renal artery can aid the clinician performing the RDN to better locate their treatment devices for a successful nerve ablation. The RDN hemodynamics co-registration system includes the fluoroscopic or angiographic view of the renal arterial tree taken during the procedure.

The present disclosure aids substantially in performing interventional procedures such as renal denervation, by improving the clinician's ability to understand where the treatment will be most effective. Implemented on a processor in communication with one or more sensors, the RDN hemodynamics co-registration system disclosed herein provides practical detection and measurement of the patient's renal nerves, their proximity to the renal artery, and their likely response to RDN treatment. This improved situational awareness transforms a blind medical procedure with a 70% success rate into one where the success can be accurately predicted and even measured in real time, to determine if more treatment is necessary, without the normally routine need to wait and see whether the patient's hypertension declines over a period of days or weeks. This unconventional approach improves the functioning of the renal denervation system, by improving the success rate of RDN treatments and by providing information necessary to perform the procedure only in locations where the renal nerve bundles are likely to be affected.

The RDN hemodynamics co-registration system may be implemented at least partially as a process viewable on a display, and operated by a control process executing on a processor that accepts user inputs from a keyboard, mouse, or touchscreen interface, and that is in communication with one or more sensors. In that regard, the control process performs certain specific operations in response to different inputs or selections made at different times. Certain outputs of the RDN hemodynamics co-registration system may be printed, shown on a display, indicated with lights or tones, or otherwise communicated to human operators. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the RDN hemodynamics co-registration system. Certain features may be added, removed, or modified without departing from the spirit of the claimed subject matter.

The present disclosure is related to U.S. Provisional Application No. _, filed _, and titled "Catheter-Based Procedures With Procedure Room Detection Of Biomarkers In Patient Blood And Associated Devices, Systems, And Methods" (Atty. Dkt. No. 2023PF00849 / 44755.2399PV01), U.S. Provisional Application No. _, filed _, and titled "Multi-Factor Renal Denervation Index For Patient Suitability and/or Expected Responsiveness To Renal Denervation Treatment" (Atty. Dkt. No. 2023PF00861 / 44755.2401PV01), U.S. Provisional Application No. _, filed _, and titled "Renal Nerve Bundle Co-Registration With X-Ray Image For Renal Denervation Treatment Guidance" (Atty. Dkt. No. 2023PF00858 / 44755.2406PV01), and U.S. Provisional Application No. _, filed _, and titled "Renal Denervation Treatment Assessment Using Ambulatory Blood Pressure Monitor" (Atty. Dkt. No. 2024PF00043 / 44755.2414PV01), each of which is incorporated by reference as though fully set forth herein.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

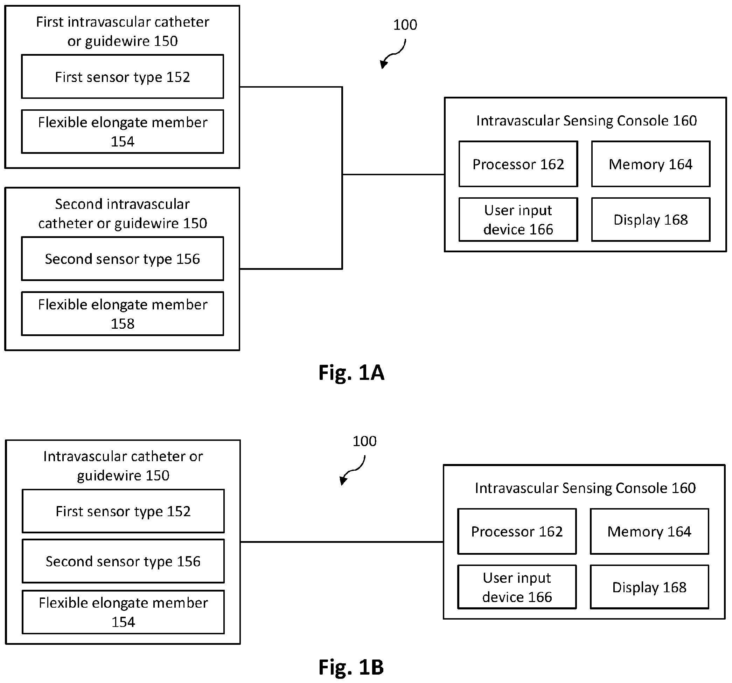

**Figure 1A** is a schematic, diagrammatic representation, in block diagram form, of an example intravascular sensing system 100, according to aspects of the present disclosure. In the example shown in Figure 1A, the intravascular sensing system 100 includes two intravascular catheters or guidewires 150. The first intravascular catheter or guidewire 150 includes a first sensor type 152 and a flexible elongate member 154. The second intravascular catheter or guidewire 150 includes a second sensor type 156 and a second flexible elongate member 158. In an example, the first intravascular catheter or guidewire 150 is a flow-sensing guidewire and the second intravascular catheter or guidewire 150 is a pressure-sensing catheter, although other arrangements are possible and fall explicitly within the scope of the present disclosure. The first intravascular catheter or guidewire 150 and the second intravascular catheter or guidewire 150 are connected to, and in electrical and data communication with, an intravascular sensing console 160. The intravascular sensing console 160 includes a processor 162, memory 164, user input device 166, and display 168.

**Figure 1B** is a schematic, diagrammatic representation, in block diagram form, of an example intravascular sensing system 100, according to aspects of the present disclosure. In the example shown in Figure 1B, the intravascular sensing system 100 includes a single intravascular catheter or guidewire 150 which includes a first sensor type 152, a second sensor type 156, and a flexible elongate member 154. In an example, the intravascular catheter or guidewire 150 is a combination pressure-sensing and flow-sensing guidewire, although other arrangements are possible and fall explicitly within the scope of the present disclosure. The intravascular catheter or guidewire 150 is connected to, and in electrical and data communication with, an intravascular sensing console 160. The intravascular sensing console 160 includes a processor 162, memory 164, user input device 166, and display 168.

Block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available.

Similarly, block diagrams may show a particular arrangement of components, modules, services, steps, processes, or layers, resulting in a particular data flow. It is understood that some embodiments of the systems disclosed herein may include additional components, that some components shown may be absent from some embodiments, and that the arrangement of components may be different than shown, resulting in different data flows while still performing the methods described herein.

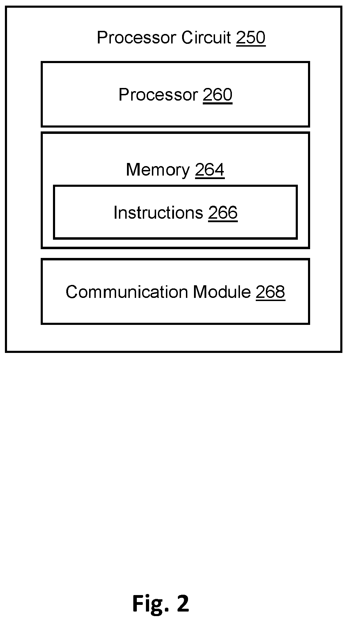

**Figure 2** is a schematic diagram of a processor circuit 250, according to aspects of the present disclosure. The processor circuit 250 may be implemented in the intraluminal sensing system 100, or other devices or workstations (e.g., third-party workstations, network routers, etc.), or on a cloud processor or other remote processing unit, as necessary to implement the method. As shown, the processor circuit 250 may include a processor 260, a memory 264, and a communication module 268. These elements may be in direct or indirect communication with each other, for example via one or more buses.

The processor 260 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 260 may also comprise another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an aspect, the memory 264 includes a non-transitory computer-readable medium. The memory 264 may store instructions 266. The instructions may be computer readable and computer executable. The instructions 266 may include instructions that, when executed by the processor 260, cause the processor 260 to perform some or all of the operations described herein. The instructions may encode some, or all, of the steps as performed by the processor circuit and as defined in the methods herein. In some embodiments the instructions encode steps of invoking execution of the steps of the method without encoding at least some of the steps such as the calculation and/or performing of co-registration themselves. Preferably, in such case, the instructions also encode steps of receiving/ or generating the display screen and for outputting the display screen to a display device. Optionally, in such case, the instructions also encode controlling a display device to output the display screen to a user. The instructions can thus be local instructions (e.g. a client program) executed on a local processor or processor circuit that invoke execution of instructions (e.g. server program) that implement the method of the invention at least partly (e.g. the calculation) or even entirely. Such invoking can be automatic, or user instigated via a user interface. The local processor or processor circuit can be separate from the processor or processor circuit. For example, the processor circuit may represent a network, or other type of electronic system that communicates with the local processor. Alternatively, the local processor can be part of (e.g. comprised within) the processor or processor circuit. Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The instructions can take the form of a computer program or computer program product.

The communication module 268 can include any electronic circuitry and/or logic circuitry to facilitate direct or indirect communication of data between the processor circuit 250, and other processors or devices. In that regard, the communication module 268 can be an input/output (I/O) device. In some instances, the communication module 268 facilitates direct or indirect communication between various elements of the processor circuit 250 and/or the intravascular sensing system 100. The communication module 268 may communicate within the processor circuit 250 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the annular ultrasound imaging array) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

It will also be understood that one or more of the steps of the methods described above can be performed by one or more components of an external imaging system, such as a processing system, a multiplexer, a beamformer, a signal processing unit, an image processing unit, or any other suitable component of the system. For example, activating the scan sequences may be carried out by a processor in communication with a multiplexer configured to select or activate one or more elements of an imaging system. The processing components of the system can be integrated within the external imaging device, contained within an external console, or may be a separate component.

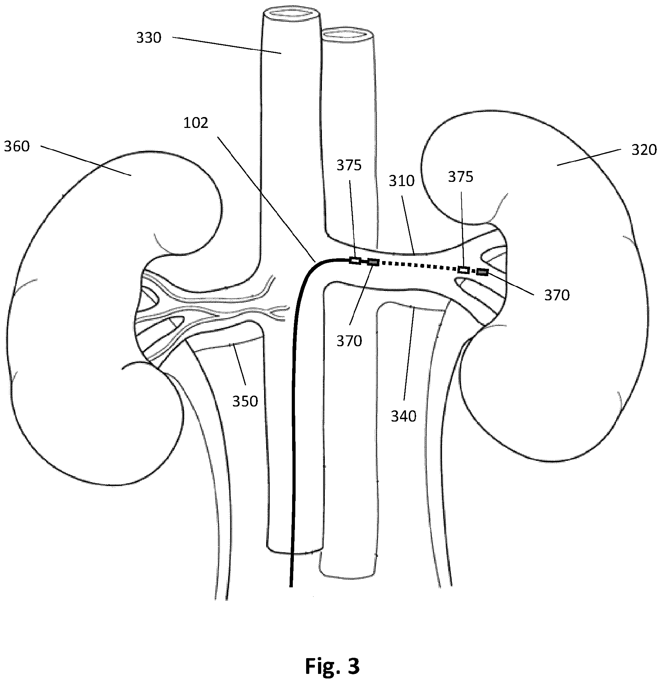

**Figure 3** is a schematic, diagrammatic representation, of an intravascular sensing guidewire 150 being pulled back through the left renal artery 310 of the left kidney 320, according to aspects of the present disclosure. The sensing components 370, 375 of the intravascular sensing guidewire can gather data (e.g., pressure and flow data) from inside the renal artery 310, thus providing information about the functioning of the renal nerves at various points along the length of the renal artery 310, that may be indicative of the success of RDN treatment at those locations.

Also visible are the abdominal aorta 330, left renal vein 340, right renal vein 350, and right kidney 360.

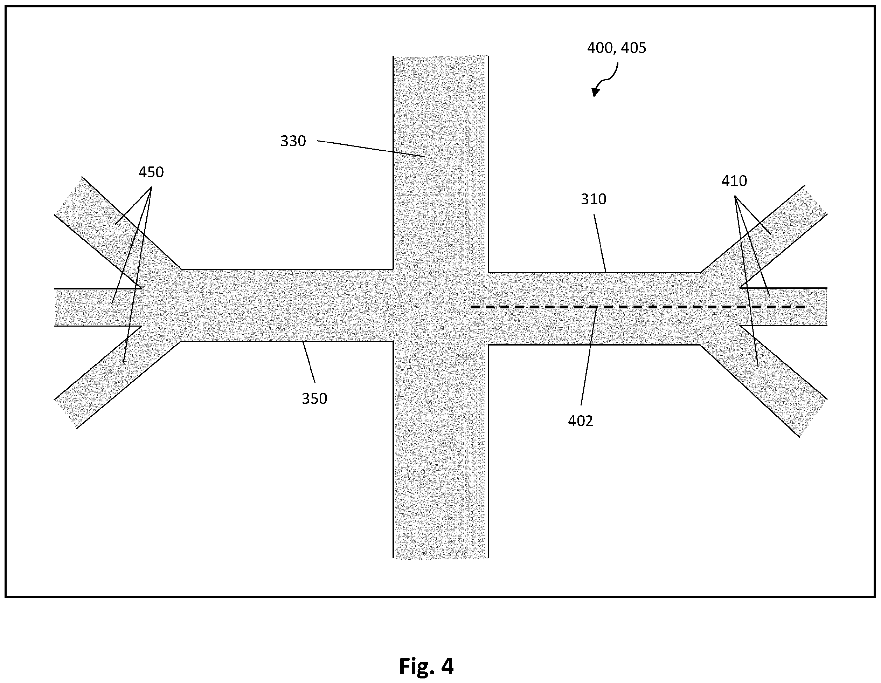

**Figure 4** is a schematic, diagrammatic representation of an X-ray image 400, with contrast, of the renal arterial tree 405, according to aspects of the present disclosure. Visible are the abdominal aorta 330, left renal artery 310, left renal artery branches 410, right renal artery 350, and right renal artery branches 450. An X-ray image with contrast may for example be taken prior to the RDN procedure to provide a roadmap of the renal arterial tree 405 to help guide the procedure. Overlaid on the renal arterial tree 405 is the pullback path 402 of the intravascular sensing device 150 of Figure 3. Figure 4 may for example be a screen display showing the renal arterial tree as a roadmap image.

In some aspects, instead of being based on an angiographic image, the roadmap image bay be a 3D model of the renal arterial tree, or a portion thereof, constructed from CT image data.

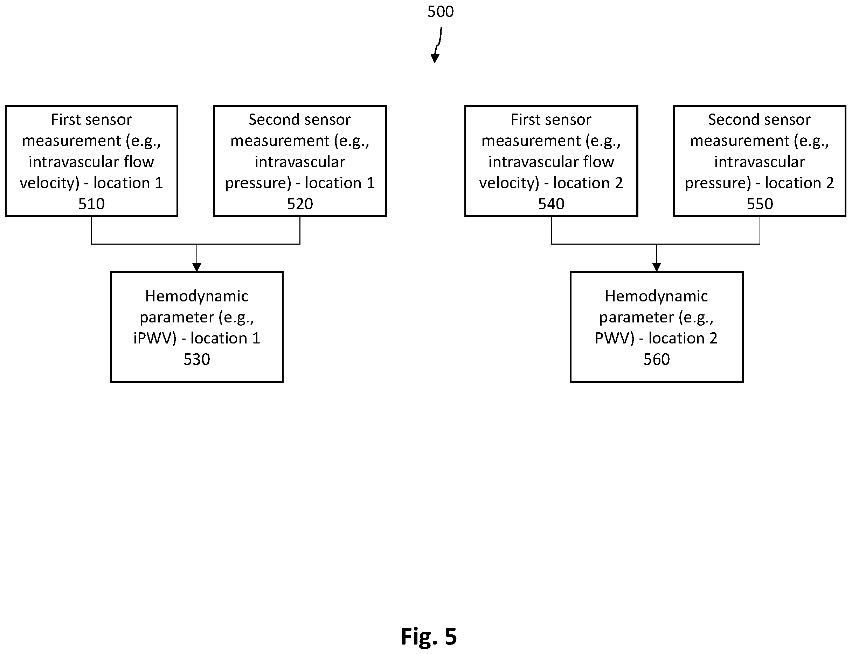

**Figure 5** is a schematic, diagrammatic representation, in block diagram form, of an example hemodynamic parameter measurement process 500, according to aspects of the present disclosure. In the example shown in Figure 5, a first sensor gathers a first measurement 510, such as intravascular flow velocity, at a first location within the renal artery, and a second sensor gathers a second measurement 520, such as intravascular pressure, at the same first location. The first measurement 510 and the second measurement 520 are then used to calculate a hemodynamic parameter 530, such as iPWV, for the first location.

Examples of pulse wave velocity calculation can be found for example in U.S. Publication No. 2019/0290139, filed May 19, 2017, U.S. Publication No. 20190175035, filed May 19, 2017, U.S. Patent No. 11,896,422, filed August 13, 2019, and U.S. Publication No. 20230233168, filed January 19, 2023, each of which is incorporated by reference as though fully set forth herein.

The hemodynamic parameter provides information about the first location that may be useful in assessing whether RDN treatment will be effective at that location. For example, an iPWV value of less than 14.4 m/s for the first location may indicate that the first location is suitable for RDN treatment, and that the patient will respond (e.g., experience a systolic blood pressure drop of at least 5 mmHg over a period of 3-6 months) to treatment at this location. It is noted that, depending on the patient and type of RDN treatment device, treatment may be needed in the main branch and the accessories. Thus, in some cases, multiple locations are needed rather than it being only a choice between different locations.

In the example shown in Figure 5, the first sensor then gathers the first measurement 510, such as intravascular flow velocity, at a second location within the renal artery, and the second sensor gathers the second measurement 520, such as intravascular pressure, at the same second location. The first measurement 510 and the second measurement 520 are then used to calculate the hemodynamic parameter 530, such as iPWV, for the second location. The hemodynamic parameter provides information about the second location that may be useful in assessing whether RDN treatment will be effective at that location. For example, if the iPWV value at the second location is lower than at the first location, then the second location may be more suitable for the RDN treatment, whereas if the iPWV value at the second location is greater than at the first location, then the second location may be less suitable for the RDN treatment. This scenario can be an example of providing guidance about choosing one treatment location between different RDN treatment locations. In another scenario, both the first and second locations may be suitable for the RDN treatment based on the measured parameters. Depending on the patient and type of RDN device, treatment may be needed in the main branch and the accessories. In other words, it is possible RDN treatment at multiple locations is needed. Guidance can be provided for the multiple locations for RDN treatment.

In the example shown in Figure 5, both the first sensor and the second sensor are intravascular. However, it is understood that at least one of the sensors may be located outside the patient's body, as for example with an external ultrasound probe measuring blood flow velocity.

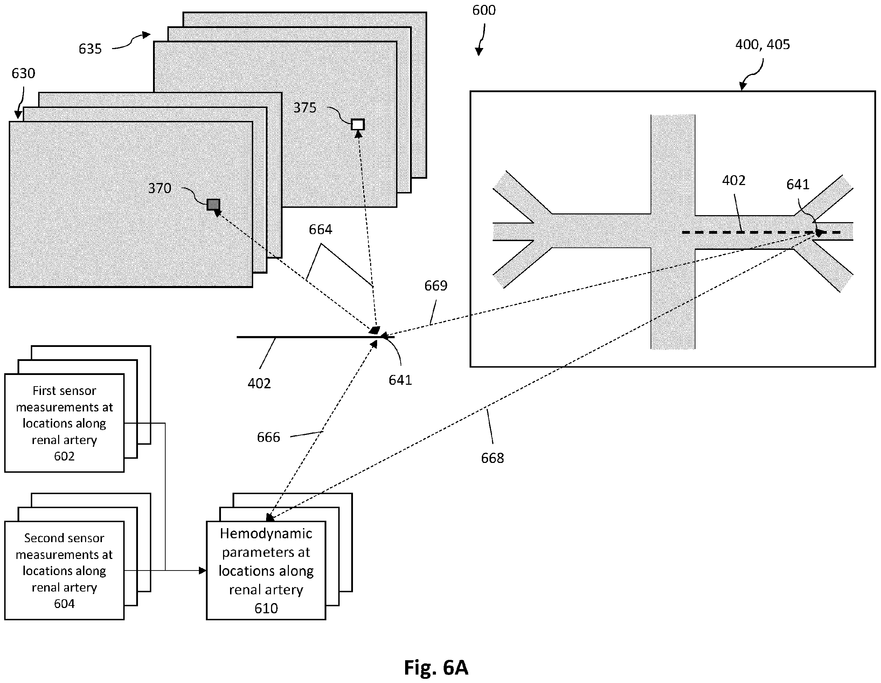

**Figure 6A** is a schematic, diagrammatic representation 600 of the co-registration of hemodynamic parameter measurements 610 with the angiographic roadmap image 400 of the renal arterial tree 405, according to aspects of the present disclosure. In the example shown in Figure 6A, the location of the first sensing element 370 is visible in frames 630 of a live fluoroscopic X-ray display taken during the RDN procedure, and the location of the second sensing element 375 is visible in frames 635 of the live fluoroscopic X-ray display. Since the first sensing element 370 and the second sensing element 375 may be in slightly different positions on the flexible elongate member, the timing of image frames 630 and 635 may be slightly different, such that they capture the sensors 370, 375 in the same locations.

Each such location is correlated with a hemodynamic parameter measurement 610, derived from the first sensor measurements 602 and second sensor measurements 604 along the pullback path 402, such that the hemodynamic measurements 610 can be added (e.g., as visual annotations) to locations of the hemodynamic measurements 610 on the pullback path 402 of the roadmap 400, along with the corresponding location of the sensor 370 or 375.

As shown by the arrows 664, because the pullback path 402 is correlated with the fluoroscopy images 630 and 635, each location 641 along the path 402 may be associated with one or more fluoroscopy images 630, 635. In addition, because a correspondence was also established between the fluoroscopy images 630, 635 and the intravascular hemodynamic measurements 610, the hemodynamic measurements 610 may also be associated with the location 741 along the path 402 as shown by the arrow 666. Just as different fluoroscopy images 630, 635 and hemodynamic parameter measurements 610 were associated with various locations along the pullback path 402, the fluoroscopy images 630, 635 and hemodynamic parameter measurements 610 may be associated with the same locations along the roadmap image 400, as shown by the arrow 668 and the arrow 669.

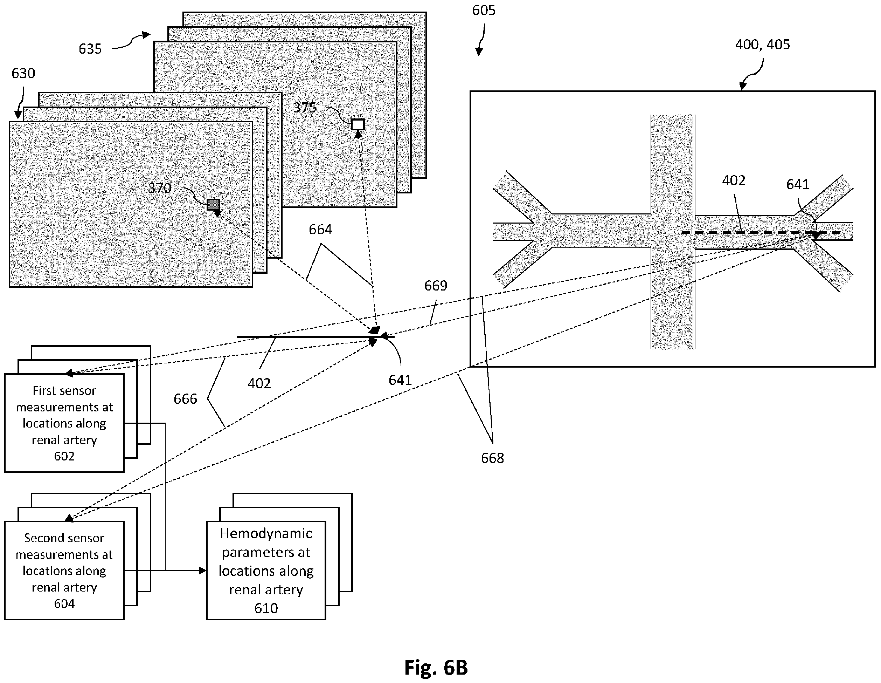

The plurality of hemodynamic parameter measurements 610 is distinct from the plurality of raw sensor measurements 602, 604. Co-registration of the sensor measurements themselves, rather than the hemodynamic parameters derived from them, is shown in Figure 6B.

**Figure 6B** is a schematic, diagrammatic representation 605 of the co-registration of raw sensor measurements 610 with the angiographic roadmap image 400 of the renal arterial tree 405, according to aspects of the present disclosure. In the example shown in Figure 6A, the location of the first sensing element 370 is visible in frames 630 of a live fluoroscopic X-ray display taken during the RDN procedure, and the location of the second sensing element 375 is visible in frames 635 of the live fluoroscopic X-ray display. Since the first sensing element 370 and the second sensing element 375 may be in slightly different positions on the flexible elongate member, the timing of image frames 630 and 635 may be slightly different, such that they capture the sensors 370, 375 in the same locations.

Each such location is correlated with sensor measurements 602, 604, along the pullback path 402, such that the sensor measurements 602, 604 can be added (e.g., as visual annotations) to locations of the sensor measurements 602, 6040 on the pullback path 402 of the roadmap 400, along with the corresponding location of the sensor 370 or 375.

As shown by the arrows 664, because the pullback path 402 is correlated with the fluoroscopy images 630 and 635, each location 641 along the path 402 may be associated with one or more fluoroscopy images 630, 635. In addition, because a correspondence was also established between the fluoroscopy images 630, 635 and the sensor measurements 602, 604, the sensor measurements 602, 604 may also be associated with the location 741 along the path 402 as shown by the arrows 666. Just as different fluoroscopy images 630, 635 and sensor measurements 602, 604 were associated with various locations along the pullback path 402, the fluoroscopy images 630, 635 and sensor measurements 602, 604 may be associated with the same locations along the roadmap image 400, as shown by the arrows 668 and the arrows 669.

Aspects of co-registration are described for example in U.S. Patent No. 7,930,014, titled "Vascular image co-registration", and U.S. Publication No. 2012/0004537, titled "Co-use of endoluminal data and extraluminal imaging", each of which is incorporated by reference as though fully set forth herein.

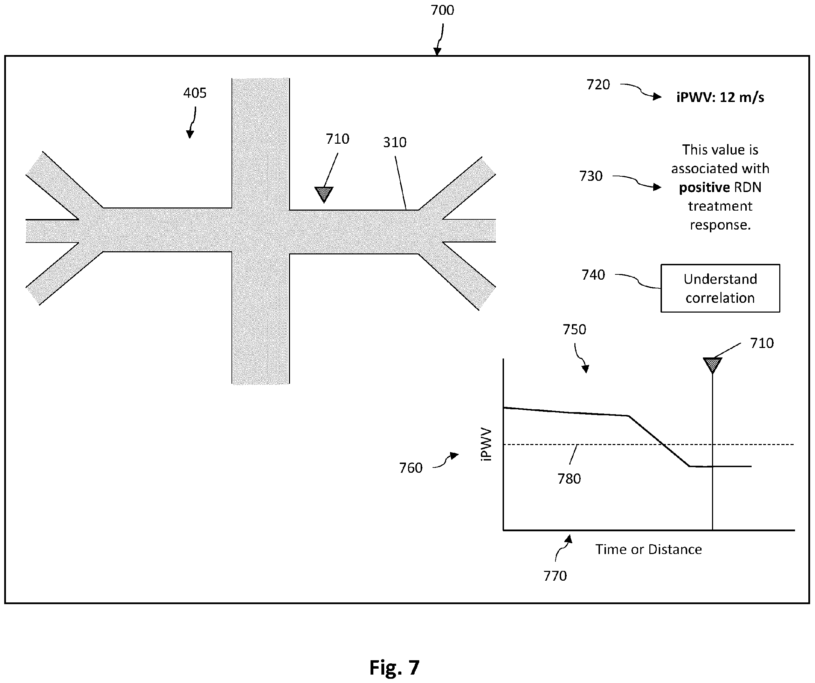

**Figure 7** is a co-registration screen display 700 of an example RDN hemodynamics co-registration system, according to aspects of the present disclosure. The screen display 700 includes a representation of the renal arterial tree 405, including a location marker 710 on the left renal artery 310, marking the position along the left renal artery 310 where the value of the hemodynamic parameter is most favorable for RDN treatment. In the example shown in Figure 7, the hemodynamic parameter 230 is invasive pulse wave velocity (iPWV), and at the location of the marker 710, it has a value 720 of 12.0 m/s. There is scientific literature that an iPWV value of 14.4 m/s or below is associated with positive RDN outcomes. The value of the iPWV may vary along the length of renal artery. Therefore, the marker 710 may for example identify a location where iPWV is below 14.4 m/s (or other cutoff value) for positive RDN outcomes, or could identify a location where iPWV has a minimum value along the length of the renal artery.

The screen display 700 also includes a text explanation 730, stating why the value 720 has been marked, and a graph 750, showing how iPWV 760 varies with time or distance 770 along the pullback. The graph 750 include a threshold line 780, indicating the cutoff value of 14.4 m/s, below which RDN treatment is more likely to be successful. The marker 710 also appears on the graph 750, so that a clinician glancing at the screen display can understand how the graph 750 related to the representation of the renal arterial tree 405. In an example, the clinician may decide to apply RDN treatment at the longitudinal location along the left renal artery 310 that is identified by the location marker 710.

The screen display 700 also includes an "Understand Correlation" button 740, the operation of which is explained below.

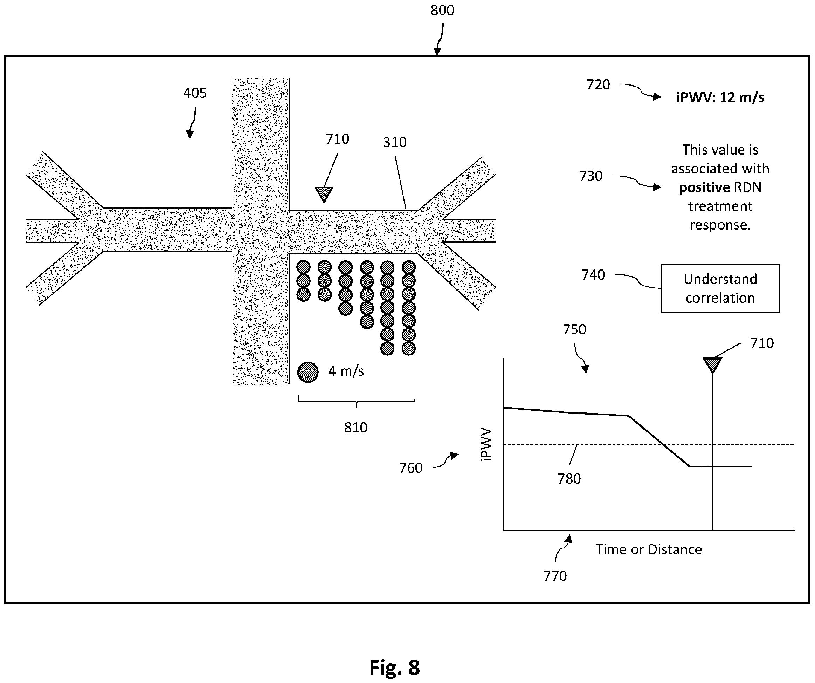

**Figure 8** is a co-registration screen display 800 of an example RDN hemodynamics co-registration system, according to aspects of the present disclosure. The screen display 800 of Figure 8 has similar features to the screen display 700 of Figure 7, except that it also includes numeric indicators 810 showing the value of the hemodynamic parameter 720 at different locations along the length of the renal artery 310. In the example shown in Figure 8, the numeric indicators 810 are dots or circles, each representing 4 meters per second, such that locations with fewer dots have a lower value for the hemodynamic parameter (e.g., iPWV), while locations with more dots have a higher value for the hemodynamic parameter. In an example, the clinician may choose to apply RDN treatment at one or more locations where the iPWV is represented by three or fewer dots (equating to a numerical value of around 12 m/s), and may avoid applying treatment to locations where the iPWV is represented by four or more dots (equating to a value of around 16 m/s or higher).

The screen display 800 also includes an "Understand Correlation" button 740, the operation of which is explained below.

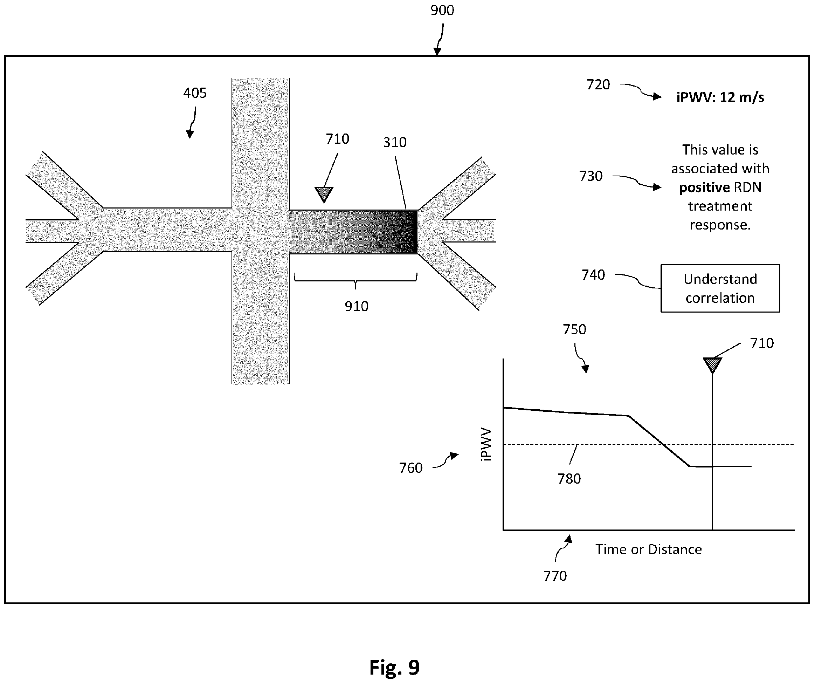

**Figure 9** is a co-registration screen display 900 of an example RDN hemodynamics co-registration system, according to aspects of the present disclosure. The screen display 900 of Figure 9 has similar features to the screen display 700 of Figure 7, except that it also includes a shading bar 910 overlaid on the renal artery 310, whose hue or shading at different locations along the renal artery 310 is representative of the value of the hemodynamic parameter 720 (e.g., iPWV) at those locations. In the example shown in Figure 9, a lighter shade is indicative of a lower value for the hemodynamic parameter 720, while a darker shade is indicative of a higher value for the hemodynamic parameter 720. In an example, the clinician may choose to apply RDN treatment to one or more areas represented by a light color, while avoiding areas represented by a dark color.

The screen display 900 also includes an "Understand Correlation" button 740, the operation of which is explained below.

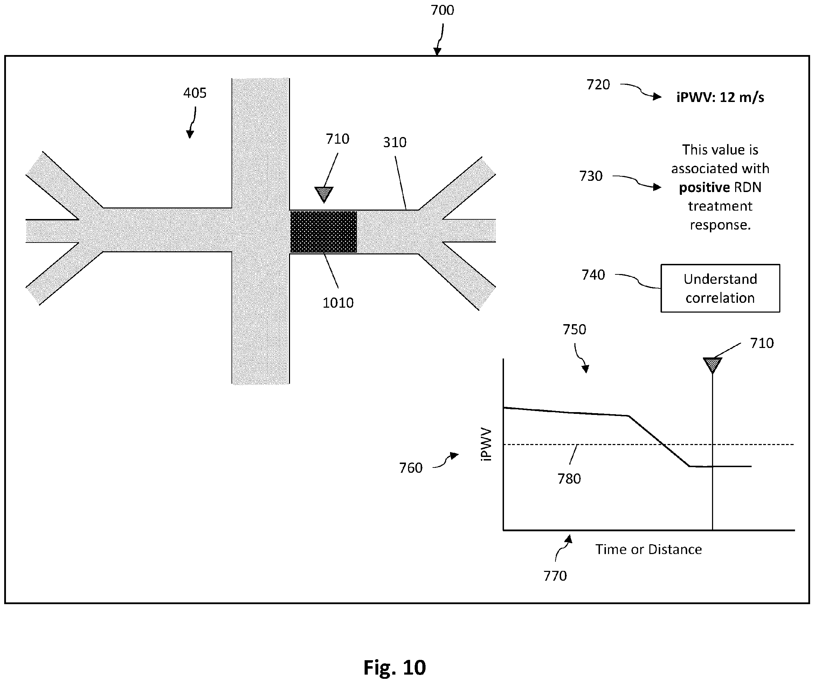

**Figure 10** is a co-registration screen display 1000 of an example RDN hemodynamics co-registration system, according to aspects of the present disclosure. The screen display 1000 of Figure 10 has similar features to the screen display 700 of Figure 7, except that it includes a threshold marker 1010 overlaid on the renal artery 310, whose presence indicates that the hemodynamic parameter at that location is above or below a specified threshold. In the example shown in Figure 10, the threshold marker 1010 indicates that for the locations covered by the threshold marker 1010, the iPWV is below the threshold of 14.4 m/s, for association with positive RDN outcomes. In an example, the clinician may choose to apply RDN treatment to one or more locations indicated by the threshold marker 1010, while avoiding locations not marked by the threshold marker 1010.

The screen display 1000 also includes an "Understand Correlation" button 740, the operation of which is explained below.

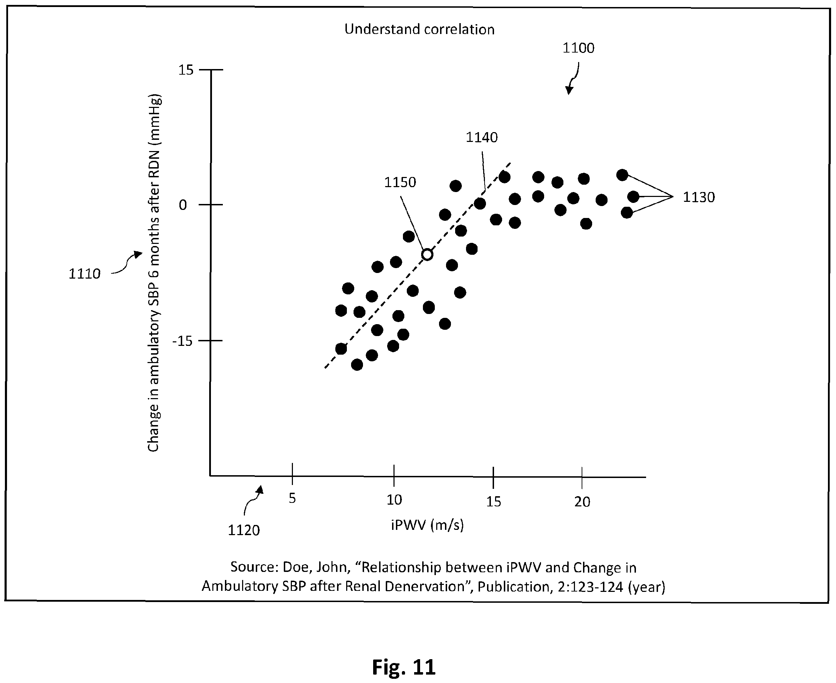

**Figure 11** is a graph 1100 showing a relationship between RDN outcomes (e.g., a measured change in ambulatory systolic blood pressure 1110 (SBP, measured in mmHg) 6 months after the RDN treatment) and the measured value of iPWV 1120 (e.g., in m/s), according to aspects of the present disclosure. The graph includes a number of historical data points 1130, a correlation-fitted line or curve 1140, and a measured value 1150 for the current patient. As can be seen in the graph 1100, there is a correlation between blood pressure reduction 1110 and iPWV 1120 for iPWV values below 14.4 m/s. The graph 1100 may for example come from scientific literature, and may be a screen display or popup window that appears when the user (e.g., a clinician) presses the "Understand Correlation" button shown in Figures 7-10.

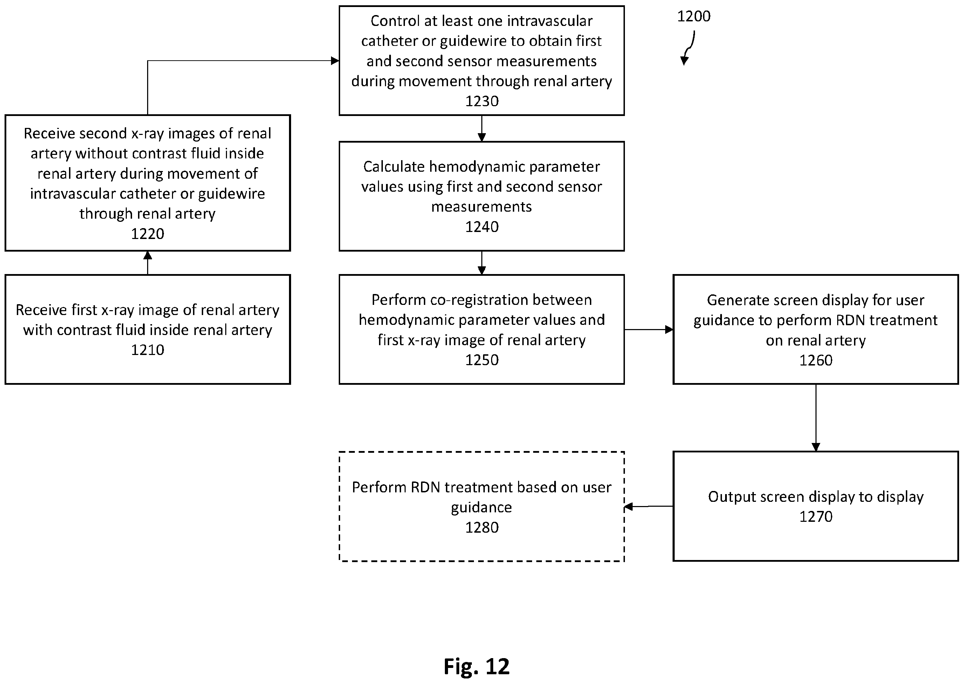

**Figure 12** is a schematic, diagrammatic representation, in flow diagram form, of an example RDN hemodynamics co-registration method 1200, according to aspects of the present disclosure. It is understood that the steps of method 1200 may be performed in a different order than shown in Figure 12, additional steps can be provided before, during, and after the steps, and/or some of the steps described can be replaced or eliminated in other embodiments. One or more of steps of the method 1200 can be carried by one or more devices and/or systems described herein, such as components of the system 100 and/or processor circuit 250.

In step 1210, the method 1200 includes receiving a first X-ray image of the renal artery with contrast fluid inside the renal artery (e.g., the roadmap image). Execution then proceeds to step 1220.

In step 1220, the method 1200 includes receiving a series of second X-ray images, without contrast fluid inside the artery (e.g., the live fluoroscopy images), during movement of the intravascular catheter or guidewire through the renal artery. Execution then proceeds to step 1230.

In step 1230, the method 1200 includes controlling at least one intravascular catheter or guidewire to obtain the first and second sensor measurements (e.g., intravascular blood flow velocity and blood pressure) during movement of the intravascular catheter or guidewire through the renal artery. Execution then proceeds to step 1240.

In step 1240, the method 1200 includes calculating the hemodynamic parameter values (e.g., iPWV values) using the first and second sensor measurements. Execution then proceeds to step 1250.

In step 1250, the method 1200 includes performing the co-registration between the hemodynamic parameter values and the first X-ray image of the renal artery (e.g., the roadmap image). Execution then proceeds to step 1260.

In step 1260, the method 1200 includes generating a screen display for user guidance to perform the RDN treatment on the renal artery. Execution then proceeds to step 1270.

In step 1270, the method 1200 includes outputting the screen display to a display (e.g., display 168 of Figure 1A or 1B). Execution then proceeds to step 1280.

In step 1280, the method 1200 includes the clinician performing the RDN treatment based on the user guidance provided in the screen display. The method 1200 is now complete.

Flow diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available.

Similarly, the logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein. For example, the measurement, co-registration, and display steps may occur in real time during an RDN procedure.

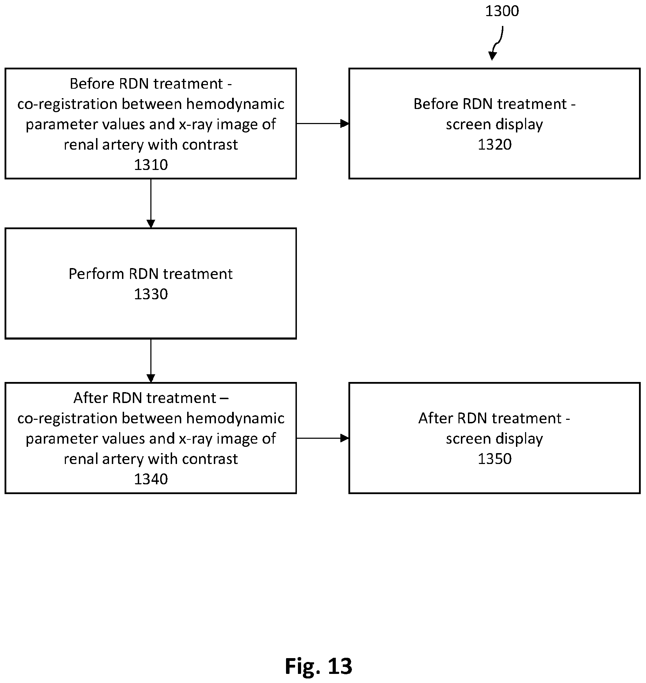

**Figure 13** is a schematic, diagrammatic representation, in flow diagram form, of an example RDN hemodynamics co-registration method 1300, according to aspects of the present disclosure.

In step 1310, the method 1300 includes, before the RDN treatment, performing the co-registration between the hemodynamic parameter values and the X-ray image of the renal artery with contrast (e.g., the roadmap image), and generating/displaying a pre-RDN-treatment screen display 1320. Execution then proceeds to step 1330.

In step 1330, the method 1300 includes the clinician performing the RDN treatment (e.g., at a location indicated as favorable by the screen display 1320). Execution then proceeds to step 1340.

In step 1340, the method 1300 includes, after the RDN treatment, performing the co-registration between the hemodynamic parameter values and the X-ray image of the renal artery with contrast (e.g., the roadmap image), and generating/displaying a post-RDN-treatment screen display 1350. In an example, changes in the values of the hemodynamic parameter along the length of the renal artery between the pre-treatment display 1320 and the post-treatment display 1350 may be indicative of the success of the RDN procedure, and may indicate whether treatment is complete or whether more treatment needs to be applied. The method 1300 is now complete.

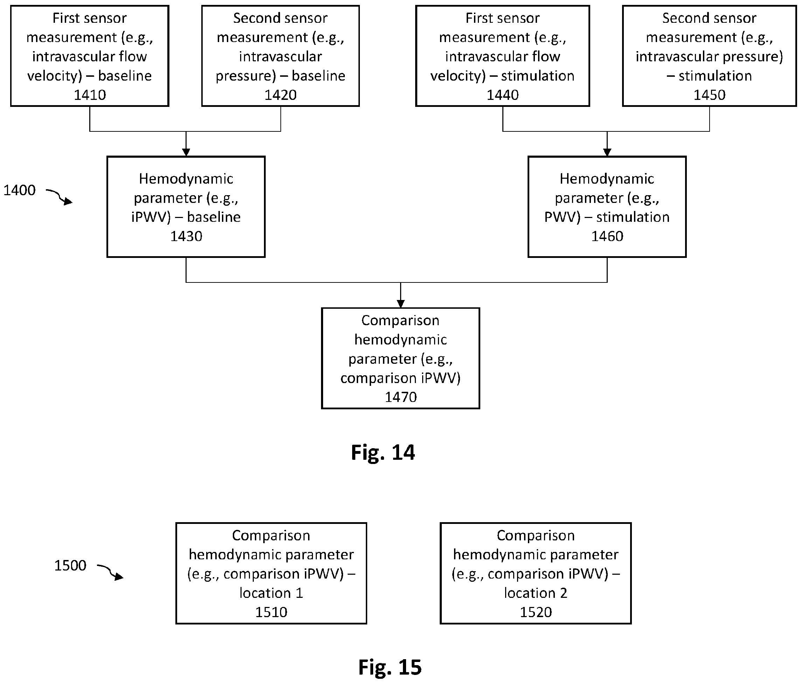

**Figure 14** is a schematic, diagrammatic representation, in block diagram form, of an example RDN hemodynamics co-registration process 1400, according to aspects of the present disclosure.

A baseline first sensor measurement 1410 (e.g., intravascular flow velocity) and a baseline second sensor measurement 1420 (e.g., intravascular pressure) are used to calculate a baseline hemodynamic parameter 1430 (e.g., iPWV). Then a stimulation is applied (e.g., a physical, electrical, pr pharmacological stimulation of the renal nerves, or of the body generally), which alters physiological parameters of the patient (e.g., by increasing heart rate and blood pressure). During this stimulation, or soon after the stimulation has taken effect, a stimulated first sensor measurement 1410 (e.g., intravascular flow velocity) and a stimulated second sensor measurement 1420 (e.g., intravascular pressure) are used to calculate a stimulated hemodynamic parameter 1430 (e.g., iPWV). Next, a comparison 1470 is generated between the baseline hemodynamic parameter and the stimulated hemodynamic parameter. This comparison may provide information about either the suitability of the patient for RDN treatment, or the success or completion status of an RDN treatment in progress. For example, if the iPWV changes with stimulation, then the patient may be suitable for RDN, whereas if the iPWV does not change with stimulation in a statistically significant way, this may indicate that sufficient ablative energy has been applied to the renal nerve(s), and further ablation is unlikely to significantly change the RDN outcome.

The comparison 1470 can be a ratio, a difference, or otherwise.

**Figure 15** is a schematic, diagrammatic representation, in block diagram form, of an example RDN hemodynamics co-registration process 1500, according to aspects of the present disclosure. In the example of Figure 15, the hemodynamic parameter comparison 1470 of Figure 14 can be performed at multiple locations. Performing a first comparison 1510 at a first location, and a second comparison 1520 at a second location, can provide location-specific information about the state of the renal nerves at that location. For example, if the hemodynamic parameter changes with stimulation at location 1 but not at location 2, this may indicate that location 1 is suitable for RDN treatment, whereas location 2 is either not suitable for RDN treatment or has been treated sufficiently, and the patient will not benefit from further ablation of the nerves in location 2.

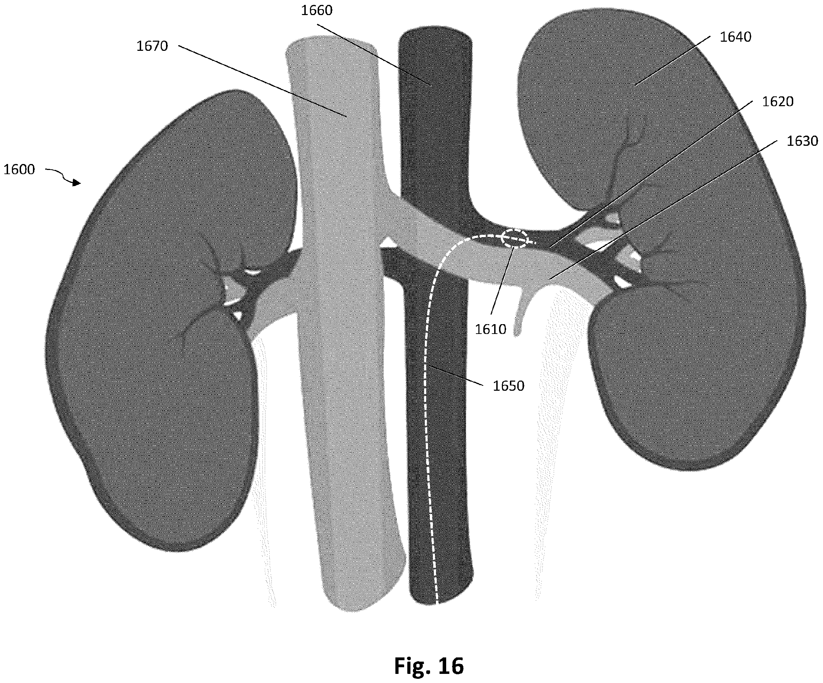

**Figure 16** is a schematic, diagrammatic representation of the renal vasculature 1600 of a patient, with a renal denervation treatment device 1610 in the left renal artery 1620 of the left kidney 1640, according to aspects of the present disclosure. In an example, the renal denervation treatment device 1610 may include a catheter 1650 with a renal denervation tool (e.g., electrodes, balloon, etc.) that delivers energy to injure the renal nerves, in order to lower the patient's blood pressure. Depending on the implementation, the delivered energy may be electrical energy, chemical energy, heat, cold, etc. The renal denervation treatment device 1610 may for example enter the renal artery 1620 via the abdominal aorta 1660. Also visible is the renal vein 1630. The renal denervation can be performed based on the guidance described herein.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an apparatus that includes a processor circuit configured for communication with at least one intravascular catheter or guidewire, where the processor circuit is configured to: control the at least one intravascular catheter or guidewire to obtain a plurality of intravascular sensor measurements associated with blood within a renal artery of a patient during movement along a length of the renal artery; calculate a plurality of hemodynamic parameter values based on the plurality of intravascular sensor measurements, where the plurality of hemodynamic parameters values is associated with at least one of a suitability of the patient for a renal denervation treatment or an expected responsiveness of the patient to the renal denervation treatment; perform co-registration between: the plurality of hemodynamic parameter values; and a first x-ray image of the renal artery obtained with a contrast fluid inside the renal artery; and output, to a display, a screen display configured to guide a user regarding the renal denervation treatment, where the screen display may include: the first x-ray image of the renal artery; and a representation of at least one hemodynamic parameter value positioned proximate to at least one corresponding location along the length of the renal artery in the first X-ray image. Other examples of this aspect include corresponding computer systems, apparatus, and computer programs recorded on one or more computer storage devices, each configured to perform the actions of the methods.

Implementations may include one or more of the following features. In some aspects, the screen display may include a graph of the plurality of hemodynamic parameters vs. time or distance. In some aspects, the screen display may include the first X-ray image overlaid with a plurality of representations of the plurality of hemodynamic parameter values, where each representation of the plurality of representations is co-located with a hemodynamic parameter value of the plurality of hemodynamic parameter values. In some aspects, each representation may include a number of dots proportional to a numerical value of a corresponding hemodynamic parameter value of the plurality of hemodynamic parameter values. In some aspects, each representation may include a color or shading corresponding to a numerical value of a corresponding hemodynamic parameter value of the plurality of hemodynamic parameter values. In some aspects, the screen display further may include a location marker indicative of a location along the renal artery. In some aspects, the location along the renal artery is a location where a corresponding hemodynamic parameter value of the plurality of hemodynamic parameter values has a minimum or maximum value. In some aspects, the location along the renal artery is a location where a corresponding hemodynamic parameter value of the plurality of hemodynamic parameter values crosses a threshold value. In some aspects, the plurality of intravascular sensor measurements may include: a plurality of first intravascular sensor measurements; and a plurality of second intravascular measurements. In some aspects, the screen display further may include a graph relating a change in blood pressure to the at least one hemodynamic parameter value. In some aspects, after the co-registration between the plurality of hemodynamic parameter values and the first x-ray image is performed, the plurality of hemodynamic parameter values is respectively associated with a plurality of locations along the length of the renal artery. In some aspects, the plurality of hemodynamic parameter values may include at least one of: invasive pulse wave velocity (iPWV), heart rate, renal resistance, or blood flow velocity. In some aspects, the processor circuit is configured to perform co-registration between the plurality of intravascular sensor measurements and the first X-ray image separate from the co-registration between the plurality of hemodynamic parameter values and the first X-ray image of the renal artery. In some aspects, to perform the co-registration between the plurality of hemodynamic parameter values and the first X-ray image of the renal artery, the processor circuit is configured to: receive the first X-ray image of the renal artery; receive a plurality of second X-ray images of the renal artery during the movement of the at least one intravascular catheter or guidewire, where the plurality of second X-ray images is obtained without the contrast fluid inside the renal artery and depicts a plurality of locations of the at least one intravascular catheter or guidewire during the movement; perform the co-registration between the plurality of hemodynamic parameter values and the first X-ray image of the renal artery using the plurality of second x-ray images. Implementations of the described techniques may include hardware, a method or process, or computer software on a computer-accessible medium.

One general aspect includes a method. The method includes controlling, with a processor circuit, at least one intravascular catheter or guidewire to obtain a plurality of intravascular sensor measurements associated with blood within a renal artery of a patient during movement along a length of the renal artery; calculating, with the processor circuit, a plurality of hemodynamic parameter values based on the plurality of intravascular sensor measurements, where the plurality of hemodynamic parameters values is associated with at least one of a suitability of the patient for a renal denervation treatment or an expected responsiveness of the patient to the renal denervation treatment; performing, with the processor circuit, co-registration between: the plurality of hemodynamic parameter values; and an x-ray image of the renal artery obtained with a contrast fluid inside the renal artery; and outputting, to a display in communication with the processor circuit, a screen display configured to guide a user regarding the renal denervation treatment, where the screen display may include: the X-ray image of the renal artery; and a representation of at least one hemodynamic parameter value positioned proximate to at least one corresponding location along the length of the renal artery in the X-ray image. Other examples of this aspect include corresponding computer systems, apparatus, and computer programs recorded on one or more computer storage devices, each configured to perform the actions of the methods.

Accordingly, it can be seen that the RDN hemodynamics co-registration system advantageously provides a means for determining, in real time during a procedure, the locations within the left or right renal artery that are most favorable for RDN treatment. The RDN hemodynamics co-registration system also provides a means to determine whether an RDN procedure is complete (e.g., whether the renal nerve bundles have been ablated sufficiently in the desired locations). The clinician may use this screen display to determine whether the RDN treatment is complete, or whether further treatment is needed to adequately ablate the renal nerve fiber bundles in the desired locations.

A number of variations are possible on the examples and aspects described above. For example, other variables may be used than those listed herein, and other sensors or sensor types may be employed. The technology described herein may be used not only before and during medical interventions, but also at other times, to provide metrics that may be indicative of a health state or disease state of the patient.

Accordingly, the logical operations making up the aspects of the technology described herein are referred to variously as operations, steps, objects, elements, components, or modules. Furthermore, it should be understood that these may occur, or be performed or arranged, in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the RDN hemodynamics co-registration system . Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the RDN hemodynamics co-registration system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

### Additional embodiments

Embodiment 1. An apparatus, comprising:
a processor circuit configured for communication with at least one intravascular catheter or guidewire, wherein the processor circuit is configured to:
control the at least one intravascular catheter or guidewire to obtain a plurality of intravascular sensor measurements associated with blood within a renal artery of a patient during movement along a length of the renal artery;
calculate a plurality of hemodynamic parameter values based on the plurality of intravascular sensor measurements, wherein the plurality of hemodynamic parameters values is associated with at least one of a suitability of the patient for a renal denervation treatment or an expected responsiveness of the patient to the renal denervation treatment;
perform co-registration between:
   the plurality of hemodynamic parameter values; and
   a first X-ray image of the renal artery obtained with a contrast fluid inside the renal artery; and
output, to a display, a screen display configured to guide a user regarding the renal denervation treatment, wherein the screen display comprises:
   the first X-ray image of the renal artery; and
   a representation of at least one hemodynamic parameter value positioned proximate to at least one corresponding location along the length of the renal artery in the first X-ray image.

Embodiment 2. The apparatus of Embodiment 1, wherein the screen display comprises a graph of the plurality of hemodynamic parameters vs. time or distance.

Embodiment 3. The apparatus of Embodiment 1, wherein the screen display comprises the first X-ray image overlaid with a plurality of representations of the plurality of hemodynamic parameter values, wherein each representation of the plurality of representations is co-located with a hemodynamic parameter value of the plurality of hemodynamic parameter values.

Embodiment 4. The apparatus of Embodiment 3, wherein each representation comprises a number of dots proportional to a numerical value of a corresponding hemodynamic parameter value of the plurality of hemodynamic parameter values.

Embodiment 5. The apparatus of Embodiment 3, wherein each representation comprises a color or shading corresponding to a numerical value of a corresponding hemodynamic parameter value of the plurality of hemodynamic parameter values.

Embodiment 6. The apparatus of Embodiment 3, wherein the screen display further comprises a location marker indicative of a location along the renal artery.

Embodiment 7 The apparatus of Embodiment 6, wherein the location along the renal artery is a location where a corresponding hemodynamic parameter value of the plurality of hemodynamic parameter values has a minimum or maximum value.

Embodiment 8. The apparatus of Embodiment 6, wherein the location along the renal artery is a location where a corresponding hemodynamic parameter value of the plurality of hemodynamic parameter values crosses a threshold value.

Embodiment 9. The apparatus of Embodiment 1, wherein the plurality of intravascular sensor measurements comprises:
a plurality of first intravascular sensor measurements; and a
a plurality of second intravascular measurements.

Embodiment 10. The apparatus of Embodiment 1, wherein the screen display further comprises a graph relating a change in blood pressure to the at least one hemodynamic parameter value.

Embodiment 11. The apparatus of Embodiment 1, wherein, after the co-registration between the plurality of hemodynamic parameter values and the first X-ray image is performed, the plurality of hemodynamic parameter values is respectively associated with a plurality of locations along the length of the renal artery.

Embodiment 12. The apparatus of Embodiment 1, wherein the plurality of hemodynamic parameter values comprises at least one of:
invasive pulse wave velocity (iPWV), heart rate, volumetric flow rate, blood flow velocity, vascular resistance or impedance, pulsatility, arterial stiffness, renal resistance, or changes in arterial diameter.

Embodiment 13. The apparatus of Embodiment 1, wherein the processor circuit is configured to perform co-registration between the plurality of intravascular sensor measurements and the first X-ray image separate from the co-registration between the plurality of hemodynamic parameter values and the first X-ray image of the renal artery.

Embodiment 14. The apparatus of Embodiment 1, wherein, to perform the co-registration between the plurality of hemodynamic parameter values and the first x-ray image of the renal artery, the processor circuit is configured to:
receive the first x-ray image of the renal artery;
receive a plurality of second X-ray images of the renal artery during the movement of the at least one intravascular catheter or guidewire, wherein the plurality of second X-ray images is obtained without the contrast fluid inside the renal artery and depicts a plurality of locations of the at least one intravascular catheter or guidewire during the movement;
perform the co-registration between the plurality of hemodynamic parameter values and the first X-ray image of the renal artery using the plurality of second x-ray images.

Embodiment 15. A method, comprising:
controlling, with a processor circuit, at least one intravascular catheter or guidewire to obtain a plurality of intravascular sensor measurements associated with blood within a renal artery of a patient during movement along a length of the renal artery;
calculating, with the processor circuit, a plurality of hemodynamic parameter values based on the plurality of intravascular sensor measurements, wherein the plurality of hemodynamic parameters values is associated with at least one of a suitability of the patient for a renal denervation treatment or an expected responsiveness of the patient to the renal denervation treatment;
performing, with the processor circuit, co-registration between:
   the plurality of hemodynamic parameter values; and
   an X-ray image of the renal artery obtained with a contrast fluid inside the renal artery; and
outputting, to a display in communication with the processor circuit, a screen display configured to guide a user regarding the renal denervation treatment, wherein the screen display comprises:
   the X-ray image of the renal artery; and
   a representation of at least one hemodynamic parameter value positioned proximate to at least one corresponding location along the length of the renal artery in the X-ray image.

## Claims

1. A processor circuit configured for communication with at least one intravascular catheter or guidewire, wherein the processor circuit is configured to:
calculate a plurality of hemodynamic parameter values based on a plurality of intravascular sensor measurements associated with blood within a renal artery of a patient obtained by at least one intravascular catheter or guidewire during movement of the at least one catheter or guidewire along a length of the renal artery, wherein the plurality of hemodynamic parameters values is associated with at least one of a suitability of the patient for a renal denervation treatment or an expected responsiveness of the patient to the renal denervation treatment;
perform co-registration between:
the plurality of hemodynamic parameter values; and
a first X-ray image of the renal artery obtained with a contrast fluid inside the renal artery; and
output, to a display, a screen display configured to guide a user regarding the renal denervation treatment, wherein the screen display comprises:
the first X-ray image of the renal artery; and
a representation of at least one hemodynamic parameter value positioned proximate to at least one corresponding location along the length of the renal artery in the first X-ray image.

2. The processor circuit of claim 1, wherein the screen display comprises a graph of the plurality of hemodynamic parameters vs. time or distance.

3. The processor circuit of claim 1 or 2, wherein the screen display comprises the first X-ray image overlaid with a plurality of representations of the plurality of hemodynamic parameter values, wherein each representation of the plurality of representations is co-located with a hemodynamic parameter value of the plurality of hemodynamic parameter values.

4. The processor circuit of claim 3, wherein each representation comprises a number of dots proportional to a numerical value of a corresponding hemodynamic parameter value of the plurality of hemodynamic parameter values.

5. The processor circuit of claim 3, wherein each representation comprises a color or shading corresponding to a numerical value of a corresponding hemodynamic parameter value of the plurality of hemodynamic parameter values.

6. The processor circuit of any one of claims 1 to 3, wherein the screen display further comprises a location marker indicative of a location along the renal artery, and wherein the location along the renal artery is a location where a corresponding hemodynamic parameter value of the plurality of hemodynamic parameter values has a minimum or maximum value.

7. The processor circuit of claim 6, wherein the screen display further comprises a location marker indicative of a location along the renal artery, and wherein the location along the renal artery is a location where a corresponding hemodynamic parameter value of the plurality of hemodynamic parameter values crosses a threshold value.

8. The processor circuit of any one of claims 1 to 7, wherein the screen display further comprises a graph relating a change in blood pressure to the at least one hemodynamic parameter value.

9. The processor circuit of any one of claims 1 to 8, wherein, after the co-registration between the plurality of hemodynamic parameter values and the first X-ray image is performed, the plurality of hemodynamic parameter values is respectively associated with a plurality of locations along the length of the renal artery.

10. The processor circuit of any one of claims 1 to 9, wherein the plurality of hemodynamic parameter values comprises at least one of:
invasive pulse wave velocity (iPWV), heart rate, volumetric flow rate, blood flow velocity, vascular resistance or impedance, pulsatility, arterial stiffness, renal resistance, or changes in arterial diameter.

11. The processor circuit of any one of claims 1 to 10, wherein the processor circuit is configured to perform co-registration between the plurality of intravascular sensor measurements and the first X-ray image separate from the co-registration between the plurality of hemodynamic parameter values and the first X-ray image of the renal artery.

12. The processor circuit of any one of claims 1 to 11, wherein, to perform the co-registration between the plurality of hemodynamic parameter values and the first x-ray image of the renal artery, the processor circuit is configured to:
receive the first x-ray image of the renal artery;
receive a plurality of second X-ray images of the renal artery during the movement of the at least one intravascular catheter or guidewire, wherein the plurality of second X-ray images is obtained without the contrast fluid inside the renal artery and depicts a plurality of locations of the at least one intravascular catheter or guidewire during the movement;
perform the co-registration between the plurality of hemodynamic parameter values and the first X-ray image of the renal artery using the plurality of second x-ray images.

13. An apparatus comprising:
- a processor circuit as claimed in any one of claims 1 to 12; and
- at least one of a display or the at least one intravascular catheter or guidewire configured to obtain a plurality of intravascular sensor measurements associated with blood within a renal artery of a patient during movement along a length of the renal artery.

14. A method, comprising:
calculating, with the processor circuit, a plurality of hemodynamic parameter values based on a plurality of intravascular sensor measurements associated with blood within a renal artery of a patient obtained by at least one intravascular catheter or guidewire during movement of the at least one intravascular catheter or guidewire along a length of the renal artery, wherein the plurality of hemodynamic parameters values is associated with at least one of a suitability of the patient for a renal denervation treatment or an expected responsiveness of the patient to the renal denervation treatment;
performing, with the processor circuit, co-registration between:
the plurality of hemodynamic parameter values; and
an X-ray image of the renal artery obtained with a contrast fluid inside the renal artery; and
outputting, to a display in communication with the processor circuit, a screen display configured to guide a user regarding the renal denervation treatment, wherein the screen display comprises:
the X-ray image of the renal artery; and
a representation of at least one hemodynamic parameter value positioned proximate to at least one corresponding location along the length of the renal artery in the X-ray image; and
optionally, controlling a display device to output the display screen to a user.

15. Computer program, comprising instructions executable by a processor or processor circuit, the instructions encoding the steps of the method of claim 14.

16. Computer program, comprising instructions executable by a processor or processor circuit, the instructions encoding invoking the steps of the method of claim 14; encoding controlling a display device to output the display screen to a user; and, optionally, encoding outputting to the display device the screen display.
